Europäisches Patentamt

European Patent Office

Office européen des brevets

(19)

(11) Numéro de publication : **0 155 427
B1**

## (12) FASCICULE DE BREVET EUROPÉEN

(45) Date de publication du fascicule du brevet :
04.05.88

(21) Numéro de dépôt : 84402677.3

(22) Date de dépôt : 20.12.84

(51) Int. Cl.⁴ : **C 12 Q 1/04, C 12 N 1/00**

(54) **Nouveau milieu de culture, solide, à délitement rapide, prêt à l'emploi et procédé pour le préparer.**

(30) Priorité : 09.02.84 FR 8402000

(43) Date de publication de la demande :
25.09.85 Bulletin 85/39

(45) Mention de la délivrance du brevet :
04.05.88 Bulletin 88/18

(84) Etats contractants désignés :
AT BE CH DE GB IT LI NL SE

(56) Documents cités :
DE-A- 2 220 065
DE-A- 2 220 065
FR-A- 2 170 923
FR-A- 2 281 068
THE OXOID MANUAL OF CULTURE MEDIA, INGRE-
DIENTS AND OTHER LABORATORY SERVICES, 3e
édition, section 1, 1969, Oxoid Ltd., pages 9-18,
Londres, GB; "Introduction,advantages and use of
oxoid media"
CHEMICAL ABSTRACTS, volume 57, no. 1, 9 juillet
1962, colonne 969b, Columbus, Ohio, US; Z.I. BRY-
KOVA et al.: "Use of sodium bicarbonate and tartaric
acid in tablets"

(73) Titulaire : **SOCIETE D'APPLICATIONS PHARMACEU-
TIQUES ET BIOLOGIQUES HOECHST-BEHRING
260 avenue Napoléon Bonaparte
F-92500 Rueil Malmaison (FR)**

(72) Inventeur : **Demiaux, Jean-Bernard
4 Rue Pierre Berteau
F-61300 L'Aigle (FR)**
Inventeur : **Gallie, Jean-Claude
Tour Mermoz L'Horloge
F-61300 L'Aigle (FR)**
Inventeur : **Spieser, Claude
46 Tour Jules Romains La Madeleine
F-61300 L'Aigle (FR)**
Inventeur : **Volle, Pierre-Jean
141 Boulevard Péreire
F-75017 Paris (FR)**

(74) Mandataire : **Orès, Bernard et al
Cabinet ORES 6, Avenue de Messine
F-75008 Paris (FR)**

## Description

La présente invention est relative à un nouveau milieu de culture, solide, à délitement rapide et prêt à l'emploi.

Dès 1909, Doerr en Allemagne avait désséché sur plaques de verre des milieux de culture pour pouvoir les conserver. Quelques années plus tard Frost (aux USA) avait effectué une étude systématique de la déssication des milieux de culture. Actuellement, les milieux solides sont préparés d'une manière industrielle. Par exemple, la maison Difco de Détroit propose un nombre considérable de milieux souvent très complexes, à l'état désséché. L'emploi de tels milieux nécessite toutefois de nombreuses manipulations : il faut peser, stériliser, répartir stérilement, dissoudre.

Ces différentes opérations sont souvent longues, certaines pouvant être plus ou moins complètes et leur succession source d'erreurs lors de dosages ultérieurs.

Il est également connu que le phénomène d'effervescence, applicable éventuellement à un comprimé, peut être obtenu par un agent constitué d'un mélange d'acide tartrique et de bicarbonate de sodium et/ou de carbonate de lysine (Chemical Abstract 57 969b, FR-A-2 281 068, FR-A-2 170 923).

La présente invention s'est par conséquent fixée pour but de pourvoir à un milieu solide, prêt à l'emploi, ne nécessitant aucune pesée, aucune manipulation de stérilisation et de répartition, d'une solubilisation parfaite, rapide et totale.

La présente invention a pour objet un milieu de culture caractérisé en ce qu'il se présente sous forme d'un comprimé comprenant tous les composants nécessaires pour effectuer l'analyse souhaitée, avec un mélange d'acide tartrique et de bicarbonate de sodium et/ou de carbonate de lysine qui entraîne l'effervescence et le délitement du comprimé quand ledit comprimé est placé dans de l'eau, et permettant l'obtention d'un milieu de culture prêt à l'emploi, en ce que le mélange d'acide tartrique et de bicarbonate de sodium et/ou de carbonate de lysine représente 13 à 22 % du poids total du comprimé, et en ce que l'association acide tartrique-bicarbonate de sodium et/ou carbonate de lysine se fait selon les proportions 1/2 et 1/4.

Ce mode de réalisation est très avantageux : dans ces conditions, au moment d'addition de l'eau de dissolution le comprimé flotte et les bulles dégagées qui peuvent être gênantes pour les mesures optiques éventuelles se regroupent par le fait même à la surface.

Conformément à l'invention, le comprimé formé d'une quantité de divers réactifs composant le milieu, quantité juste nécessaire et suffisante pour une opération analytique unitaire, le poids dudit comprimé étant compris entre 0,01 g et 5 g.

Selon un mode de réalisation avantageux de l'objet de l'invention, le comprimé peut être rendu stérile.

Le comprimé selon l'invention peut contenir si on le désire, un réactif d'inhibation de croissance de tout microorganisme (bactéries, virus, champignon, levure...) à différentes concentrations.

Selon un autre mode de réalisation, le comprimé selon l'invention peut contenir des indicateurs de croissance (indicateurs d'oxydo-réduction, indicateur de pH, indicateurs colorés de croissance, etc).

Selon encore un autre mode de réalisation de l'objet de l'invention, le comprimé peut contenir des réactifs d'identification des caractéristiques métaboliques (tels que par exemple des substrats enzymatiques mannitol, lactose, etc).

Conformément à l'invention, le comprimé contient en outre, un ou des agent(s) lubrifiant(s) qui peut par ailleurs être un stabilisant (du benzoate de sodium et/ou du P.E.G. 6000 par exemple).

Les avantages de tels Milieux de culture solides sont remarquables : ils peuvent être tamponnés au pH désiré, optimal pour une croissance donnée : ils sont immédiatement, tels quels, prêts à l'emploi, très maniables et d'une solubilité rapide et totale ; ils conviennent aussi bien à la croissance de bactéries, virus, champignons, levures, qu'à la croissance cellulaire.

La présente invention a également pour objet un procédé de préparation du milieu solide conforme à la présente invention, caractérisé en ce que l'on procède à une pluralité de prémélanges successifs de tous les constituants du milieu avant d'effectuer la compression et, éventuellement la stérilisation.

Selon un mode de réalisation avantageux du procédé objet de la présente invention, un, plusieurs ou la totalité des constituants entrant dans la composition du milieu sont séchés par lyophilisation.

L'invention pourra être mieux comprise à l'aide du complément de description qui va suivre, qui se réfère à des exemples de compositions et de préparation du milieu conforme à la présente invention.

Il doit être bien entendu, toutefois, que les différents exemples de mise en œuvre qui seront décrits ci-après, sont donnés uniquement à titre d'illustration de l'objet de l'invention, mais n'en constituant en aucune manière une limitation.

(Voir Tableau I page 3)

2

**0 155 427**

Exemple I. Comprimé contenant un antibiotique

Tableau I

| | Minima | Maxima |
|---|---|---|
| Antibiotique | 0,0003 | 10 |
| Milieu de culture lyophilisé contenant l'antibiotique | 0,6 | 90 |
| Acide tartrique | 3,33 | 15 |
| Bicarbonate de Sodium | 6,67 | 40 |
| Benzoate de Sodium | 0 | 10 |
| Milieu de culture | 50 | 95 |

Les quantités sont indiquées en pourcentage (poids sur poids).

Exemple II. Composition de comprimés contenant de la Gentamycine à différentes concentrations.

Tableau II

| GENTAMYCINE | 0,000333 | 0,000666 | 0,001333 | 0,00266 | 0,005333 | 0,01066 | 0,02133 |
|---|---|---|---|---|---|---|---|
| MILIEU DE CULTURE LYOPHILISE CONTENANT LA GENTAMYCINE | 0,624 | 1,248 | 2,496 | 4,992 | 9,984 | 19,968 | 39,936 |
| ACIDE TARTRIQUE | 4,333 | 4,333 | 4,333 | 4,333 | 4,333 | 4,333 | 4,333 |
| BICARBONATE DE SODIUM | 10,666 | 10,666 | 10,666 | 10,666 | 10,666 | 10,666 | 10,666 |
| BENZOATE DE SODIUM | 5,000 | 5,000 | 5,000 | 5,000 | 5,000 | 5,000 | 5,000 |
| MILIEU DE CULTURE | 79,386 | 78,773 | 77,493 | 75,000 | 70,000 | 60,000 | 40,000 |

Les quantités sont indiquées en pourcentage.

0 155 427

Exemple III. Composition de comprimés contenant de la Gentamycine à des concentrations allant de 0,125 μg à 8 μg par comprimé et de raison 2.

Tableau III

| GENTAMYCINE | 0,125 | 0,250 | 0,500 | 1 | 2 | 4 | 8 |
|---|---|---|---|---|---|---|---|
| MILIEU DE CULTURE LYOPHILISE CONTENANT LA GENTAMYCINE | 234 | 469 | 938 | 1 875 | 3 750 | 7 500 | 15 000 |
| ACIDE TARTRIQUE | 1 625 | 1 625 | 1 625 | 1 625 | 1 625 | 1 625 | 1 625 |
| BICARBONATE DE SODIUM | 4 000 | 4 000 | 4 000 | 4 000 | 4 000 | 4 000 | 4 000 |
| BENZOATE DE SODIUM | 1 875 | 1 875 | 1 875 | 1 875 | 1 875 | 1 875 | 1 875 |
| MILIEU DE CULTURE | 29 766 | 29 531 | 29 062 | 28 125 | 26 250 | 22 500 | 15 000 |
| POIDS D'UN COMPRIME | 37 500 | 37 500 | 37 500 | 37 500 | 37 500 | 37 500 | 37 500 |

Les quantités sont indiquées en microgrammes.

0 155 427

**0 155 427**

Exemple IV. Composition centésimale de milieux de culture

Milieu 1

| | |
|---|---:|
| Bio-trypcase | 39,58 |
| Bio-soyase | 13,19 |
| Chlorure de sodium | 10,55 |
| Bicarbonate de sodium | 10,55 |
| Sulfite de sodium | 0,53 |
| Acide tartrique | 4,29 |
| Cystine | 1,85 |
| Glucose | 14,51 |
| Benzoate de sodium | 4,95 |
| | 100,00 |

Milieu 2

| | |
|---|---:|
| Peptone de caséine | 45,33 |
| Peptone de farine de soja | 8,00 |
| Glucose | 6,67 |
| Chlorure de sodium | 13,33 |
| Bicarbonate de sodium | 10,67 |
| Acide tartrique | 4,33 |
| Phosphate dipotassique | 6,67 |
| Benzoate de sodium | 5,00 |
| | 100,00 |

Milieu 3

| | |
|---|---:|
| Bio-trypcase | 37,13 |
| Bio-soyase | 12,38 |
| Chlorure de sodium | 9,90 |
| Sulfite de sodium | 0,50 |
| Acide tartrique | 4,93 |
| Cystine | 1,73 |
| Glucose | 13,61 |
| Benzoate de sodium | 2,47 |
| P.E.G. 6000 | 2,48 |
| Carbonate de lysine | 14,87 |
| | 100,00 |

## Exemple V

Exemple de préparation d'un mélange destiné à la fabrication de comprimés de milieu de culture renfermant de la Ticarcilline dosée de 4 μg à 64 μg par comprimé.

On prépare un mélange contenant 16 μg d'antibiotique dans 1,875 mg.

Pour garantir une répartition homogène de l'antibiotique dans le milieu de culture, on opère par trois prémélanges successifs dans un mélangeur rapide (par exemple Lödige, Stephan, Henschel).

## Exemple

0,500 gramme d'antibiotique + 5 grammes de milieu de culture ; on mélange 30 secondes en mélangeur rapide.

On additionne à ce premier mélange 25 grammes du même milieu de culture et on mélange 30 secondes environ en mélangeur rapide.

On additionne à ce deuxième mélange 28,61 grammes du même milieu de culture ; on mélange 30 secondes environ en mélangeur rapide.

Le mélange obtenu contient 16 μg d'antibiotique dans 1,875 mg.

Le tableau IV suivant indique les quantités de ce mélange qui entrent dans la composition de comprimés titrant respectivement 4 μg, 8 μg, 16 μg, 32 μg, 64 μg de Ticarcilline par comprimé terminé de 37,5 mg.

Les quantités sont indiquées en mg.

6

# 0 155 427

Tableau IV

| : Mélange contenant l'antibiotique | 0,469 | 0,938 | 1,875 | 3,750 | 7,500 |
|---|---|---|---|---|---|
| Acide tartrique | 1,625 | 1,625 | 1,625 | 1,625 | 1,625 |
| Bicarbonate de sodium | 4,000 | 4,000 | 4,000 | 4,000 | 4,000 |
| Benzoate de sodium | 1,875 | 1,875 | 1,875 | 1,875 | 1,875 |
| Milieu de culture | 29,540 | 29,000 | 28,125 | 26,250 | 22,500 |

Il résulte de la description qui précède que quels que soient les modes de mise en œuvre, de réalisation et d'application adoptés, l'on obtient des milieux solides qui présentent par rapport aux milieux antérieurement connus des avantages importants, dont certains ont déjà été mentionnés dans ce qui précède, et notamment :

— le comprimé, très maniable et très soluble, peut être destiné à la reconstitution de milieux de culture solides, semi-solides ou liquides ;

— le comprimé est particulièrement adapté pour contenir par exemple les antibiotiques ou les antifongiques à différentes concentrations ;

— le comprimé peut être préparé à partir de tout milieu de culture pour bactéries, levures, champignons, virus et cellules ;

— le comprimé permet l'utilisation d'un système de lecture optique dans d'excellentes conditions et de ce fait permet la réalisation de toute technique microbiologique et notamment la détection et la numération à partir d'un échantillon quelconque : sérum, urine, liquide cephalo-rachidien, liquide de ponction, produit laitier, eau, etc... ;

— le comprimé à délitement rapide permet la reconstitution de milieux prêts à l'emploi pour la détermination d'un antibiogramme, d'une concentration minimale inhibitrice, d'une identification et d'une caractérisation du microorganisme par étude différentielle de croissance ;

— le comprimé conforme à la présente invention, grâce à sa parfaite et rapide solubilité permet de réaliser un milieu de culture (pour bactéries, virus, levures, champignons... et cellules) prêt à l'emploi dans des délais très brefs par simple addition d'eau distillée.

Ainsi que cela ressort de ce qui précède, l'invention ne se limite nullement à ceux de ses modes de mise en œuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1. Milieu de culture caractérisé en ce qu'il se présente sous forme d'un comprimé comprenant tous les composants nécessaires pour effectuer l'analyse souhaitée, avec un mélange d'acide tartrique et de bicarbonate de sodium et/ou de carbonate de lysine qui entraîne l'effervescence et le délitement du comprimé quand ledit comprimé est placé dans de l'eau, et permettant l'obtention d'un milieu de culture prêt à l'emploi, en ce que le mélange d'acide tartrique et de bicarbonate de sodium et/ou de carbonate de lysine représente 13 à 22 % du poids total du comprimé, et en ce que l'association acide tartrique-bicarbonate de sodium et/ou carbonate de lysine se fait selon les proportions 1 :2 à 1 :4.

2. Milieu de culture selon la revendication 1, caractérisé en ce qu'il est formé d'une quantité de divers réactifs composant le milieu, quantité juste nécessaire et suffisante pour une opération analytique unitaire, le poids dudit comprimé étant compris entre 0,01 g et 5 g.

3. Milieu de culture selon l'une quelconque des revendications 1 et 2, caractérisé en ce qu'il est rendu stérile.

4. Milieu de culture selon l'une quelconque des revendications 1 à 3, caractérisé en ce qu'il contient un réactif d'inhibition de croissance de tout microorganisme (bactéries, virus, champignon, levure...) à différentes concentrations.

5. Milieu de culture selon l'une quelconque des revendications 1 à 4, caractérisé en ce qu'il contient des indicateurs de croissance (indicateurs d'oxydo-réduction, indicateur de pH, indicateurs colorés de croissance).

6. Milieu de culture selon l'une quelconque des revendications 1 à 5, caractérisé en ce qu'il contient des réactifs d'identification des caractéristiques métaboliques (tels que des substrats enzymatiques, mannitol, lactose).

7. Milieu de culture selon l'une quelconque des revendications 1 à 6, caractérisé en ce qu'il contient, en outre, un ou des agent(s) lubrifiant(s), notamment un stabilisant (du benzoate de sodium et/ou du P.E.G. 6000).

7

8. Procédé de préparation du milieu de culture selon l'une quelconque des revendications 1 à 7, caractérisé en ce que l'on procède à une pluralité de prémélanges successifs de tous les constituants du milieu de culture avant d'effectuer la compression et, éventuellement, la stérilisation.

9. Procédé selon la revendication 8, caractérisé en ce qu'un, plusieurs ou la totalité des constituants entrant dans la composition du milieu, sont séchés par lyophilisation.

**Claims**

1. Culture medium characterized in that it is in the form of a tablet comprising all the components necessary for performing the desired analysis, with a mixture of tartaric acid and of sodium bicarbonate and/or lysine carbonate which results in effervescence and the disintegration of the tablet when the said tablet is placed in water, and enabling the production of a ready-foruse culture medium, wherein the mixture of tartaric acid and the mixture of sodium bicarbonate and/or of lysine carbonate represents 13 — 22 % of the total weight of the tablet, and wherein the association of tartaric acid — sodium bicarbonate and/or lysine carbonate is made in the proportions 1 :2 to 1 :4.

2. Culture medium according to claim 1, characterized in that it is formed of a quantity of various reagents composing the medium, said quantity being just necessary and sufficient for a single analytical operation, the weight of said tablet being comprised between 0,1 g and 0,5 g.

3. Culture medium according to any one of claims 1 and 2, characterized in that it is made sterile.

4. Culture medium according to any one of claims 1-3, characterized in that it contains a reagent for inhibiting the growth of any microorganisms (bacteria, viruses, mould, yeast...) at different concentrations.

5. Culture medium according to any one of claims 1-4, characterized in that it contains growth indicators (oxido-reduction, pH indicator, dyed growth indicators).

6. Culture medium according to any one of claims 1-5, characterized in that it contains reagents for the identification of the metabolic characteristics (such as enzymatic substrates, mannitol, lactose).

7. Culture medium according to any one of claims 1-6, characterized in that it contains, in addition, one or more lubricants, particularly a stabiliser, (sodium benzoate and/or P.E.G. 6000).

8. Process for the preparation of the culture medium according to any one of claims 1-7, characterized in that a plurality of successive premixes is made of all the constituents of the culture medium before performing the compression and, possibly, sterilization.

9. Process according to claim 8, characterized in that one, several or the totality of the constituents entering into the composition of the medium, are dried by freeze-drying.

**Patentansprüche**

1. Kulturmedium, dadurch gekennzeichnet, daß es in Form einer Tablette vorliegt, die alle zur Durchführung der gewünschten Analyse notwendigen Bestandteile umfaßt, mit einem Gemisch aus Weinsäure und Natriumbicarbonat und/oder Lysincarbonat ; was ein Sprudeln und Zerfallen der Tablette bewirkt, wenn diese in Wasser gelegt wird und es erlaubt, ein zur Verwendung fertiges Kulturmedium zu erhalten, wobei das Gemisch aus Weinsäure und Natriumbicarbonat und/oder Lysincarbonat 13 bis 22 Gew.-% des Gesamtgewichts der Tablette ausmacht und wobei das Verhältnis von Weinsäure zu Natriumbicarbonat und/oder Lysincarbonat im Bereich von 1 :2 bis 1 :4 liegt.

2. Kulturmedium nach Anspruch 1, dadurch gekennzeichnet, daß es aus einer solchen Menge von verschiedenen Reagenzien, welche das Medium bilden, besteht, die für eine einheitliche Analyse gerade erforderlich und ausreichend ist, wobei das Gewicht der genannten Tablette zwischen 0,01 g und 5 g liegt.

3. Kulturmedium nach Anspruch 1 und 2, dadurch gekennzeichnet, daß es steril gemacht worden ist.

4. Kulturmedium nach einem der Ansprüche 1 bis 3, dadurch gekennzeichnet, daß es ein wachstumsinhibierendes Reagens für alle Mikroorganismen (Bakterien, Viren, Pilze, Hefe) bei verschiedenen Konzentrationen enthält.

5. Kulturmedium nach einem der Ansprüche 1 bis 4, dadurch gekennzeichnet, daß es Wachstumsindikatoren (Redox-Indikatoren, pH-Indikatoren, gefärbte Wachstumsindikatoren) enthält.

6. Kulturmedium nach einem der Ansprüche 1 bis 5, dadurch gekennzeichnet, daß es Reagenzien zur Bestimmung metabolischer Eigenschaften (wie enzymatische Substrate, Mannit, Lactose) enthält.

7. Kulturmedium nach einem der Ansprüche 1 bis 6, dadurch gekennzeichnet, daß es zusätzlich ein oder mehrere Schmiermittel, insbesondere einen Stabilisator (Natriumbenzoat und/oder P.E.G. 6000) enthält.

8. Verfahren zur Herstellung eines Kulturmediums nach einem der Ansprüche 1 bis 7, dadurch gekennzeichnet, daß man vor dem Verpressen und gegebenenfalls Sterilisieren mit einer Mehrzahl von aufeinanderfolgenden Vorgemischen aus allen Bestandteilen des Kulturmediums verfährt.

9. Verfahren nach Anspruch 8, dadurch gekennzeichnet, daß ein, mehrere oder die gesamten Bestandteile, die in die Zusammsetzung des Mediums eingearbeitet werden, durch Lyophilisierung getrocknet werden.